# EUROPÄISCHE PATENTSCHRIFT

(11) **EP 0 273 141 B1**
(45) Veröffentlichungstag und Bekanntmachung des Hinweises auf die Patenterteilung: **27.01.1993**
(21) Anmeldenummer: 87116249.1
(22) Anmeldetag: 04.11.1987
(51) Int. Cl.: A61L 15/00

(54) **Superabsorberfaserflocken, Verfahren zu ihrer Herstellung und ihre Verwendung**
Superabsorbent fibre flocks, method for making them and their use
Flocons fibreux superabsorbants, leur procédé de fabrication et leur utilisation

(30) Priorität: 08.12.1986 DE 3641893; 21.09.1987 DE 8712723 U
(43) Veröffentlichungstag der Anmeldung: 06.07.1988
(73) Patentinhaber: Steen & Co. GmbH, D-22508 Hamburg (DE)
(72) Erfinder: Knack, Ingo, Dr., D-2053 Schwarzenbek (DE); Beckert, Wolfgang, D-2073 Lütjensee (DE)
(74) Vertreter: Lederer, Franz, Dr.

(56) Entgegenhaltungen:
- EP-A- 0 047 009
- EP-A- 0 156 257
- EP-A- 0 174 775
- DE-A- 2 846 593
- DE-A- 3 002 136
- US-A- 3 901 236

## Beschreibung

Polymerprodukte mit hohem Absorptionsvermögen finden ihre Anwendung in Bereichen, in denen wäßrige Flüssigkeiten entfernt und an eine feste Phase gebunden werden sollen. Bevorzugte Anwendung finden derartige Produkte bei der Herstellung von Hygieneprodukten, die die Aufgabe haben, wäßrige Flüssigkeiten, wie beispielsweise Blut oder Urin aufzusaugen, damit der Träger des Hygieneproduktes nicht durch ein unangenehmes Gefühl der Feuchtigkeit in seinem Wohlbefinden beeinträchtigt wird.

Es sind bereits pulverförmige Granulate eines schwach vernetzten Polymerisates bekannt, die in die Hygieneprodukte, die neben dem saugfähigen Polymer eine Matrix-Struktur aufweisen, die im allgemeinen aus einem Zellstoffgewebe besteht, eingearbeitet werden. Diese Matrix-Struktur wird auch als Fluffschicht bezeichnet.

Es hat sich herausgestellt, daß bei der Herstellung der Hygieneartikel eine richtige Dosierung des saugaktiven Polymerproduktes nur sehr schwer zu erreichen ist, da das Polymerprodukt bei der Herstellung der Hygieneartikel durch die sich bildende Matrix-Struktur (Fluffschicht) hindurch wieder abgesaugt wird.

Es wurde weiterhin beobachtet, daß beim Verpacken und beim Transport der Endprodukte das saugaktive Polymerprodukt eine starke Migration oder Delokalisation erfährt, wodurch die Produkte eine unerwünschte Formdeformation erleiden können oder ein Teil des saugaktiven Polymerproduktes bei der Verwendung nicht mehr zur Verfügung steht.

Thermisch verbundene Polyethylen-Faserpulpen für Absorptionsprodukte, wie beispielsweise Windeln werden von der Firma Hercules, Wilmington, USA unter dem Warenzeichen Pulpex (R) auf den Markt gebracht. Die thermisch verbundene Polyethylenfasernetzstruktur soll Superabsorberpulver besser zurückhalten.

Eine Holzfluffpulpe, die durch Verschmelzen einer Polyolefinpulpe gehalten wird, wird in der EP-A1 123 414 und der korrespondierenden US-P 4 458 042 beschrieben.

Ein Hygieneartikel enthaltend ein superwasserabsorbierendes Polymeres, das zwischen zwei Schichten aus Zellstoff-Flocken angeordnet ist, wird in der DE-OS 35 11 805 beschrieben. Durch Veränderung der Packungsdichte soll die Absorptionskapazität erhöht werden.

Die DE-OS 36 17 881 beschreibt einen absorbierenden Artikel aus einem Basismaterial aus Fasern, die zumindest teilweise hydrophob sind, und einem wasserabsorbierenden Polymeren, welches zumindest teilweise in Form von, im wesentlichen kugelförmigen Partikeln vorliegt und mit den Fasern, diese umgebend, verbunden ist. Die polymeren Partikeln sind durch das Fasermaterial verstreut und an die Fasern in der Form von sphärischen oder ellipsoiden Perlen gebunden die nacheinander aufgereiht sind, etwa perlschnurartig in der Form eines Rosenkranzes. Dieser bekannte Artikel wird hergestellt, indem man eine wäßrige Lösung eines wasserlöslichen ethylenisch ungesättigten Monomeren auf das aus den hydrophoben Fasern bestehende Basismaterial aufbringt, so daß ein Teil der Lösungstropfen sich um die Fasern herumlegt und an diese bindet, das Monomere polymerisiert zur Erzeugung des wasserabsorbierenden Polymeren und den sich hieraus ergebenden Artikel trocknet. Der Artikel behält also die Form des aus Fasern bestehenden Basismaterials bei, in welchen sich einzelne Perlen des wasserabsorbierenden Polymeren befinden. Leicht handhabbare Flocken, die zu Artikeln unterschiedlicher Formgebung verarbeitet werden können, entstehen so nicht.

Mit Fasern bedeckte Hydrogel-Partikel werden in der US-PS 3 901 236 beschrieben, wobei hydrophile Fasern bevorzugt eingesetzt werden. Eine Verbindung zwischen Fasern und Hydrogel wird dadurch erzielt, daß das Hydrogel, vorzugsweise mit Wasser, erweicht wird und die Fasern dann an dem Hydrogel haften.

Aufgabe der vorliegenden Erfindung war es ein hochsaugfähiges, teilchenförmiges Polymerprodukt zur Verfügung zu stellen, das ähnlich einem Pulver gehandhabt werden kann, aber dennoch fest innerhalb der Fluffschicht von Hygieneartikeln haftet, wodurch die Nachteile der bekannten Polymerprodukte vermieden werden.

Das erfindungsgemäße Polymerprodukt weist eine hohe Absorptionsfähigkeit und eine Teilchenstruktur auf, bei der viele kurze Fasern in den einzelnen Polymerpartikeln eingeschlossen sind, wobei insbesondere die Enden der kurzen Fasern aus den Polymerpartikeln herausragen. Durch die herausragenden Fasernteile bzw. Fasernenden können die erfindungsgemäßen Polymerprodukte mit der Fluffschicht in Wechselwirkung treten, so daß bei der Einarbeitung eine lokale Fixierung der einzelnen Polymerprodukte in der Matrix erreicht werden kann. Das Absorberpolymer liegt also in Form vieler kleiner Partikel vor, die jeweils das Zentrum der Faserflocken bilden.

Gegenstand der Errindung sind somit Superabsorberfaserflocken bestehend aus einem Kern aus einem saugfähigen Polymer in den kurze Polymerfasern eingelagert sind, die zumindest teilweise aus dem Kern herausragen, wobei der Kern aus mittels eines Haftvermittlers miteinander verbundenen Partikeln des saugfähigen Polymers und den Polymerfasern besteht.

Das erfindungsgemäß verwendete saugfähige Polymermaterial muß in der Lage sein, eine möglichst große Menge einer wäßrigen Flüssigkeit absorbieren zu können. In der Regel werden dazu bevorzugt Polymerisate auf der Basis von Acrylsäure/Natriumacrylat verwendet. Aber auch andere Polymerisate auf der Grundlage von vernetzten Polymeren können im Rahmen der vorliegenden Erfindung verwendet werden. Beispiele für derartige Polymere sind quervernetzte Polysaccharide, wie beispielsweise Carboxymethylcellulose oder Stärkeether, und anorganische, poröse Materialien, wie beispielsweise Zeolite.

Die Partikeln des saugfähigen Polymers weisen im allgemeinen eine Partikelgröße von 1-1000 Mikron, vorzugsweise 100-630 Mikron auf.

In der Regel muß das verwendete Polymermaterial ein Mehrfaches seines Gewichtes an Wasser aufnehmen können; das bevorzugt verwendete Acrylsäure/Natriumacrylat-Polymer kann dabei mindestens die dreifache Menge seines Gewichtes an Wasser aufnehmen, wobei das Polymer in einen gelartigen Zustand übergeht.

Ein im Rahmen der vorliegenden Erfindung besonders bevorzugtes Polyacrylat-Produkt ist das im Handel erhältliche FAVOR® SAB 922 (Firma Stockhausen). Es weist die folgenden chemischen Daten auf:

| | |
|---|---|
| Chemische Basis: | Natrium-Polyacrylat |
| Beschaffenheit: | weißes Granulat |
| Korngröße: | 100-630 Mikron |
| Schüttgewicht: | 690 ± 30 g/l |
| Rieselverhalten: | frei fließend |
| Trockensubstanz: | 95 ± 2 % |
| pH-Wert: (0,1 % Gel in 0,9 % NaCl) | 6,0 ±1 |
| Lagerung: | bei trockener Lagerung 1 Jahr |

Dieses Natrium-Polyacrylat ist ein schwach vernetztes Polymerisat auf der Basis Acrylsäure/Natriumacrylat.

Dieses bevorzugte Polymermaterial quillt bei Wasseraufnahme zu einem weitgehend noch trockenen und körnigen Gel ohne zu zerfallen.

Als Polymerfasern werden erfindungsgemäß bevorzugt Polyolefinfasern, insbesondere Polyethylen (PE-) und Polypropylen (PP-) -Kurzschnittfasern verwendet. Diese Fasern werden nach an sich bekannten Verfahren hergestellt und weisen bevorzugt eine Länge zwischen 0,2 mm und 10 mm und besonders bevorzugt zwischen 0,5 mm und 4 mm auf. Im allgemeinen weisen die Fasern einen Titer von 0,5-6 dtex auf, wobei ein Titer von 0,7-4 dtex bevorzugt wird. Eine leichte Längenabweichung der einzelnen Fasern stört bei der erfindungsgemäßen Verwendung nicht. Es kann sogar vorteilhaft sein, gezielt unterschiedliche Faserlängen einzusetzen, um bevorzugte Haftwirkungen des Polymerproduktes in der Fluffschicht zu erzielen.

Im Rahmen der vorliegenden Erfindung können auch Fasern aus Polyester und Polyacrylnitril verwendet werden, jedoch sind die genannten PE- und PP-Kurzschnittfasern bevorzugt, da diese Fasern aufgrund ihrer molekularen Struktur eine Dochtwirkung entfalten, so daß sie die Flüssigkeit von dem Entstehungsort zu den hochsaugfähigen Polymerprodukten weiterleiten können. Zur Verbesserung der Haftfähigkeit des faserhaltigen hochsaugaktiven Polymerproduktes kann es vorteilhaft sein, die Fasern einer speziellen Behandlung zu unterziehen, durch die die Struktur der Fasern derart verändert wird, daß diese leicht gekräuselt sind und somit eine bessere mechanische Interaktion mit den Fasern der Matrix-Struktur, in die die erfindungsgemäßen Polymerpartikel eingebettet sind, zu erzielen. Ein derartiger zusätzlicher Behandlungsschritt kann das Recken der Fasern vor dem entsprechenden Ablängen sein.

Zur Herstellung der erfindungsgemäßen Produkte können prinzipiell zwei Wege beschritten werden.
a) Das Polymerprodukt mit hoher Absorptionsfähigkeit wird mit den Fasern vermischt und die Verbindung durch Zusatz eines Haftvermittlers erzielt. Die Art des verwendeten Haftvermittlers hängt sowohl von der Art des eingesetzten Polymerproduktes, wie auch von der Art der eingesetzten Fasern ab. Aufgrund der chemischen Zusammensetzung des absorptionsfähigen Polymermaterials und der Fasern kann der Fachmann diejenigen Haftvermittler auswählen, durch die eine zuverlässige Verbindung zwischen den beiden oben genannten Komponenten erzielt werden kann. Darüber hinaus dürfen die Haftvermittler natürlich keine unerwünschten Nebenwirkungen aufweisen, die von der Art der Verwendung abhängen. Sollen die Polymerprodukte beispielsweise in Hygieneartikeln, wie Windeln verwendet werden, müssen die Haftvermittler gesundheitlich unbedenklich sein.
   Unter der großen Vielzahl der bekannten Haftvermittler werden im Rahmen der vorliegenden Erfindung Ethylen/Acrylamid-Copolymere, Polymere-Isocyanate oder reaktive Silicium-organische Verbindungen bevorzugt, wobei Ethylen/Acrylsäure-Copolymere besonders bevorzugt sind.
b) Ein anderer Weg, das Fasermaterial mit dem Polymerprodukt in Verbindung zu bringen, besteht darin, daß das Fasermaterial vor der Fertigstellung des Polymermaterials in die Polymerlösung zugegeben wird und daß anschließend die Vernetzungsreaktion stattfindet. Dabei werden die Fasern, zumindest teilweise in das Polymerprodukt eingeschlossen, so daß sich eine Verbindung der Faserenden mit dem Inneren des Polymerproduktes ergibt. Durch eine derartige Herstellungsweise kann ein Produkt erzielt werden, bei dem sich die Enden der Fasern im Inneren des Polymers befinden, wodurch eine schnellere und effektivere Übermittlung der zu entfernenden Flüssigkeit in das Polymer erzielt wird, da die Fasern, wie bereits erwähnt eine Dochtwirkung aufweisen.
   Bei dieser Art der Herstellung kann eine Öl-in-Wasser-Polymerisation eingesetzt werden.

Das Verhältnis der eingesetzten Fasern zu dem hochsaugaktiven Polymerprodukt kann in relativ weiten Bereichen im allgemeinen im Bereich von 10:1 bis 1:10 verändert werden. Bevorzugt wird ein Gewichtsverhältnis von Fasern zu hochsaugaktivem Polymerprodukt von 1:1 bis 1:10 und insbesondere von 1:2 bis 1:8. Auf diese Weise lassen sich Produktvariationen gewinnen, die von einem mehr watteähnlichen, flockigen bis zu einem im wesentlichen kornartigen Polymerprodukt reichen können.

Nach dem Verkleben der Polymerkörner untereinander und mit den eingesetzten Fasern werden kleinere bis mittelgroße Faserflocken erhalten, die in einer Schlagmühle zerkleinert und anschließend getrocknet werden.

Das fertige Produkt enthält dann viele kleine Fasern in den einzelnen teilchenförmigen Polymerpartikeln eingeschlossen, die insbesondere mit ihren Enden aus diesen herausragen.

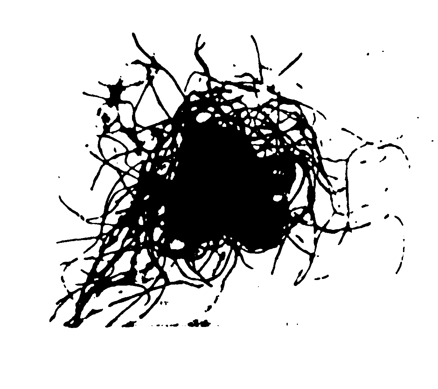

In der Abbildung wird eine erfindungsmäße Superabsorberfaserflocke gezeigt. Der Kern aus einem saugfähigen Polymer und die herausstehenden Polymerfasern sind deutlich ersichtlich.

Durch die erfindungsgemäßen faserhaltigen Polymerprodukte mit hohem Absorptionsvermögen wird die Herstellung der Endprodukte, insbesondere wenn es sich um Hygieneprodukte handelt, deutlich erleichert.

Aufgrund der erfindungsgemäßen Struktur können die erfindungsgemäßen Polymerprodukte bei der Herstellung der Hygieneprodukte gezielt dosiert werden, da eine wirkungsvolle Wechselwirkung des erfindungsgemäßen Produktes mit der Matrix des Hygieneproduktes erfolgt. Ein unerwünschtes Herausfallen bzw. Hindurchgesaugtwerden des saugfähigen Polymerproduktes aus bzw. durch das fertige Produkt kann also durch die Struktur des neuen Produktes verhindert werden. Da also das eingesetzte Material nahezu vollständig in dem fertigen Material verbleibt, kann die Dosierung entsprechend eingestellt werden und somit können unerwünschte Verluste vermieden werden.

Das fertige Hygieneprodukt muß, bis es an den Ort des endgültigen Einsatzes gelangt, verschiedene Transport- und Lagerphasen durchlaufen. Dabei soll sich natürlich die Form und Zusammensetzung des fertigen Produktes nicht verändern. Gerade durch die Struktur des erfindungsgemäßen faserhaltigen Polymerproduktes mit hoher Absorptionsfähigkeit kann eine relativ feste Verbindung des Polymerproduktes mit der anderen Komponente des Hygieneproduktes, nämlich der Matrix-Struktur erzielt werden.

Durch die Dochtwirkung der bevorzugt eingesetzten Fasern wird der Bereich eines jeden Polymerpartikels vergrößert, der aktiv die wäßrige Flüssigkeit absorbieren kann. Durch die Erfindung wird es möglich, die im Handel in Pulverform erhältlichen Superabsorberprodukte so zu verarbeiten, daß die mit der Pulverstruktur verbundenen Nachteile ausgeschaltet werden. Die erfindungsgemäßen Superabsorberflocken können Zellulose-Fluff ersetzen und kombinieren und verbessern die Absorptionsqualitäten der Superabsorberpolymeren und von Fluff mit der Migrationsstabilität von Fluff und der leichten Handhabung und Dosierungsmöglichkeit von Superabsorberpulvern.

Auch aus medizinischer Sicht weisen die erfindungsgemäßen Produkte einen Vorteil gegenüber den bekannten Produkten auf. Aufgrund der leichten Beweglichkeit der faserlosen Polymerprodukte können diese nicht nur bei der Lagerung oder dem Transport aus dem fertigen Produkt entweichen, sondern auch bei der Verwendung am menschlichen Körper können diese Polymerprodukte aus dem Hygieneprodukt austreten und dann mit der menschlichen Haut bzw. den empfindlichen Schleimhäuten in Kontakt geraten und Reizungen oder immunologisch bedingte Irritationen hervorrufen. Die erfindungsgemäßen Produkte dagegen sind fest mit der Fluffschicht des Hygieneproduktes verbunden und können deshalb nicht in direkten Kontakt mit der menschlichen Haut gelangen.

Den erfindungsgemäßen Polymerprodukten können auch antimikrobiell wirksame Stoffe, wie beispielsweise fungizid wirkende Substanzen zugesetzt werden, um dadurch einen Pilzbefall, der gerade bei Säuglingen ein schwerwiegendes Problem darstellen kann, zu verhindern.

Bevorzugt werden die erfindungsgemäßen Polymerprodukte bei der Herstellung von Hygieneprodukten und Inkontinenzartikeln verwendet. Es handelt sich hierbei bevorzugt um Babywindeln, Damenbinden, Slipeinlagen und Windeln für Erwachsene.

Eine weitere Verwendungsmöglichkeit der erfindungsgemäßen Polymerprodukte ist bei der Entfernung von Wasser aus hydrophoben Flüssigkeiten möglich. Dabei werden die erfindungsgemäßen Faserflocken in eine Filter-Matrix eingebettet und die zu filtrierende, wasserhaltige hydrophobe Flüssigkeit wird durch das Filtersystem geleitet. Bei den hydrophoben Flüssigkeiten kann es sich um organische Lösungsmittel oder auch um Treibstoffe, beispielsweise Kerosin, Dieselöl oder um Hydrauliköl handeln.

### Ausführungsbeispiele:

### Ausgangsmaterialien:

Hochsaugaktives Polymerprodukt (Marke Favor SAB 922); pulverförmiges Natriumpolyacrylat mit einer Korngröße von 100-630 Mikron.

Polyethylen (PE)- und Polypropylen (PP)-Fasern (Steen) mit einem Titer von 2,0 bzw. 2,8 dtex und einer Schnittlänge von 2 mm.

Haftvermittler: Dispersionslösung (Primacor 4983 der Fa. Dow Chemical) mit einem Gehalt von 25 % eines Ethylen/Acrylsäure-Copolymers. Verdünnung: 20 ml der 25 %igen Dispersion mit dest. Wasser auf 100 ml.

### Beispiel 1:

3 g PE-Faser (2,8 dtex, 2 mm Schnittlänge) und 6 g Polymerpulver wurden in ein 250 ml Becherglas gegeben, 6,8 g der verdünnten Dispersionslösung zugegeben und sofort durch kräftiges Rühren mit einem Glasstab gut vermischt. Durch Verkleben der Polymerkörner untereinander und mit den Fasern wurden kleinere bis mittelgroße Faserflocken erhalten, die anschließend in einer Schlagmühle zerkleinert wurden (ca. 30-60 sec. lang gemahlen).

Nach Trocknen des Produktes bei leicht erhöhter Temperatur (50-60 °C) erhielt man ein weiches, flockiges Produkt.

### Beispiel 2:

2,0 g PE-Faser (Titer 2,8 dtex, Schnittlänge 2 mm) 6 g Polymerpulver und 4,7 g der verdünnten Dispersion wurden wie unter Beispiel 1 beschrieben, verarbeitet.
- Ergebnis:: lockeres, flockiges, mehr körniges Produkt.

## Patentansprüche

1. Superabsorberfaserflocken bestehend aus einem Kern aus einem saugfähigen Polymer in den kurze Polymerfasern eingelagert sind, die zumindest teilweise aus dem Kern herausragen, dadurch gekennzeichnet daß, die Polymerfasern Polyolefin-, Polyester- oder Polyacrylnitrilfasern sind und der Kern aus mittels eines Haftvermittlers miteinander verbundenen Partikeln des saugfähigen Polymers und den Polymerfasern besteht.

2. Superabsorberfaserflocken gemäß
Ansprüch 1, dadurch gekennzeichnet, daß das saugfähige Kernpolymer ein Acrylsäure/Natriumacrylat-Polymer ist.

3. Superabsorberfaserflocken gemäß Anspruch 1,
dadurch gekennzeichnet, daß das saugfähige Kernpolymer eine Carboxymethylcellulose oder ein Stärkeether ist.

4. Superabsorberfaserflocken gemäß einem der Ansprüche 1 bis 3, dadurch gekennzeichnet, daß die Polymerfasern Polyethylen- oder Polypropylen-Kurzschnittfasern sind.

5. Superabsorberfaserflocken gemäß einem der vorgehenden Ansprüche, dadurch gekennzeichnet, daß die Polymerfasern eine Länge von 0,2 - 10 mm, vorzugsweise 0,5 - 4 mm aufweisen.

6. Superabsorberfaserflocken gemäß einem der Ansprüche 1 - 5, dadurch gekennzeichnet, daß der Haftvermittler ein Ethylen/Acrylsäure-Copolymer ist.

7. Superabsorberfaserflocken gemäß einem der Ansprüche 1 - 6, dadurch gekennzeichnet, daß die Partikeln des saugfähigen Polymers eine Partikelgröße von 1 - 1000, vorzugsweise 100 - 630 Mikron aufweisen.

8. Superabsorberfaserflocken gemäß einem der vorgehenden Ansprüche, dadurch gekennzeichnet, daß das Gewichtsverhältnis von Polymerfasern zu saugfähigem Polymer 10:1 bis 1:10, vorzugsweise 1:1 bis 1:10, insbesondere 1:2 bis 1:8 beträgt.

9. Verfahren zur Herstellung der Superabsorberfaserflocken gemäß einem der Ansprüche 1 - 8 , dadurch gekennzeichnet, daß Partikeln eines saugfähigen Polymers miteinander und mit den Polymerfasern mit Hilfe eines Haftvermittlers verbunden werden und die so erhaltenen Faserflocken auf die gewünschte Teilchengröße vermahlen werden.

10. Verfahren zur Herstellung der Superabsorberfaserflocken gemäß einem der Ansprüche 1 - 9 , dadurch gekennzeichnet, daß im Zuge der Herstellung der Partikeln eines saugfähigen Polymers vor der Vernetzungsreaktion der Polymerlösung die Polymerfasern zugesetzt werden, anschließend die Vernetzungsreaktion initiiert wird und nach der Aushärtung der Polymerpartikeln, die so erhaltenen Faserflocken auf die gewünschte Teilchengröße vermahlen werden.

11. Verwendung der Superabsorberfaserflocken gemäß einem der Ansprüche 1 - 8 in Hygieneprodukten, insbesondere Babywindeln, Damenbinden und Inkontinenzartikeln.

12. Verwendung der Superabsorberfaserflocken gemäß einem der Ansprüche 1 - 8 zur Abtrennung von Wasser und wäßrigen Bestandteilen aus hydrophoben Flüssigkeiten.

13. Verwendung der Superabsorberfaserflocken gemäß einem der Ansprüche 1 - 8 im Gartenbau und in der Landwirtschaft für Pflanzenbewässerungssysteme.

## Claims

1. Superabsorbent fibre flocks comprising a core composed of an absorbent polymer into which are embedded short polymer fibres which project at least partially from the core, characterised in that the polymer fibres are polyolefine-, polyester- or polyacrylonitrile fibres, and the core is composed of particles, connected to one another by means of a bonding agent, of the absorbent polymer and the polymer fibres.

2. Superabsorbent fibre flocks as claimed in Claim 1, characterised in that the absorbent core polymer is an acrylic acid / sodium acrylate polymer.

3. Superabsorbent fibre flocks as claimed in Claim 1, characterised in that the absorbent core polymer is a carboxymethylcellulose or a starch ether.

4. Superabsorbent fibre flocks as claimed in one of Claims 1 to 3, characterised in that the polymer fibres are polyethylene- or polypropylene short fibres.

5. Superabsorbent fibre flocks as claimed in one of the preceding claims, characterised in that the polymer fibres possess a length of 0.2 - 10 mm, preferably 0.5 - 4 mm.

6. Superabsorbent fibre flocks as claimed in one of Claims 1 - 5, characterised in that the bonding agent is an ethylene / acrylic acid copolymer.

7. Superabsorbent fibre flocks as claimed in one of Claims 1 to 6, characterised in that the particles of the absorbent polymer possess a particle size of 1 - 1000, preferably 100 - 630 microns.

8. Superabsorbent fibre flocks as claimed in one of the preceding claims, characterised in that the weight ratio of polymer fibres to absorbent polymer amounts to 10:1 to 1:10, preferably 1:1 to 1:10, in particular 1:2 to 1:8.

9. A process for the production of the superabsorbent fibre flocks claimed in one of Claims 1- 8, characterised in that particles of an absorbent polymer are connected to one another and to the polymer fibres with the aid of a bonding agent, and the fibre flocks obtained in this way are ground to the desired particle size.

10. A process for the production of the superabsorbent fibre flocks as claimed in one of Claims 1 - 9, characterised in that in the course of the production of the particles of an absorbent polymer, prior to the cross-linking reaction the polymer fibres are added to the polymer solution, whereupon the cross-linking reaction is initiated and following the hardening of the polymer particles the fibre flocks obtained in this way are ground to the desired particle size.

11. The use of the superabsorbent fibre flocks as claimed in one of Claims 1 - 8 in hygiene products, in particular baby napkins, sanitary towels and incontinence articles.

12. The use of the superabsorbent fibre flocks as claimed in one of Claims 1 - 8 for separating water and aqueous constituents from hydrophobic liquids.

13. The use of the superabsorbent fibre flocks as claimed in one of Claims 1 - 8 in horticulture and agriculture for plant watering systems.

## Revendications

1. Flocons fibreux superabsorbants constitués d'un noyau en polymère absorbant dans lequel sont insérées de courtes fibres de polymère qui dépassent au moins en partie du noyau, caractérisés en ce que les fibres de polymères sont des fibres de polyoléfine, de polyester ou de polyacrylonitrile, et le noyau est constitué des fibres de polymères et de particules du polymère absorbant liées entre elles au moyen d'un agent de pontage.

2. Flocons fibreux superabsorbants selon la revendication 1 caractérisés en ce que le polymère absorbant du noyau est un polymère acide acrylique / acrylate de sodium.

3. Flocons fibreux superabsorbants selon la revendication 1 caractérisés en ce que le polymère absorbant du noyau est une cellulose carboxyméthyle ou un éther d'amidon.

4. Flocons fibreux superabsorbants selon l'une des revendications 1 à 3 caractérisés en ce que les fibres de polymères sont des fibres courtes de polyéthylène ou de polypropylène.

5. Flocons fibreux superabsorbants selon l'une des revendications précédentes, caractérisés en ce que les fibres polymères présentent une longueur de 0,2-10 mm, de préférence 0,5-4 mm.

6. Flocons fibreux superabsorbants selon l'une des revendications 1 - 5, caractérisés en ce que l'agent de pontage est un copolymère éthylène/acide acrylique.

7. Flocons fibreux superabsorbants selon l'une des revendications 1 - 6, caractérisés en ce que les particules du polymère absorbant présentent une taille de particules de 1-1000, de préférence 100-630 microns.

8. Flocons fibreux superabsorbants selon l'une des revendications précédentes, caractérisés en ce que le rapport en poids des fibres de polymères au polymère absorbant se monte à 10:1 à 1:10 de préférence 1:1 à 1:10, en particulier 1:2 à 2:8.

9. Procédé de fabrication des flocons fibreux superabsorbants selon l'une des revendications 1 - 8 caractérisé en ce que des particules d'un polymère absorbant sont liées entre elles et avec des fibres de polymères au moyen d'un agent de pontage, et les flocons de fibres ainsi obtenus sont moulus à la taille de particules souhaitée.

10. Procédé de fabrication des flocons fibreux superabsorbants selon l'une quelconque des revendications 1 - 9 caractérisé en ce que les libres de polymères sont ajoutées au cours de la fabrication des particules de polymère absorbant avant la réaction de réticulation puis la réaction de réticulation est initiée et après durcissement des particules de polymère les flocons fibreux ainsi obtenus sont moulus à la taille de particules souhaitée.

11. Utilisation des flocons fibreux superabsorbants selon l'une des revendications 1 - 8 pour des articles d'hygiène, en particulier des couches pour bébé, des protections féminines et des articles d'incontinence.

12. Utilisation des flocons fibreux superabsorbants selon l'une des revendications 1 - 8 pour séparer l'eau et les composantes aqueuses de fluides hydrophobes.

13. Utilisation des flocons fibreux superabsorbants selon une des revendications 1 - 8 en horticulture et en agriculture pour des systèmes d'irrigation des plantes.
